# EUROPEAN PATENT APPLICATION

(11) **EP 4 659 798 A1**
(43) Date of publication of application: **10.12.2025**
(21) Application number: 25164467.0
(22) Date of filing: 18.03.2025
(51) Int. Cl.: A61N 1/40

(54) **TAILOR-MADE REMOTE CELL-ACTIVATION SYSTEM AND ITS APPLICATION METHOD**

(30) Priority: 05.06.2024 TW 113120731
(71) Applicant: Baichao Greenergy Tech. Co., Ltd., Taichung City 408 (TW)
(72) Inventor: HU, Chui-Ping, Taichung City 408 (TW); CHIANG, Chen-Ying, Taichung City 408 (TW); YEN, Chun-Tai, Taichung City 408 (TW)
(74) Representative: Viering, Jentschura & Partner mbB Patent- und Rechtsanwälte

(57) **Abstract**

A remote cell-activation system (2) and its application method are configured to work with a computer (31) or a smartphone (32) capable of internet access, sound recording, photographing, executing mobile apps, and updating applications. The remote cell-activation system (2) is developed to use quasi-like quantum entanglement pair technology to send transformed electric field information of three generated electromagnetic fields to a target object (21) in a wireless and distance-unlimited manner, so a user is free from buying specialized equipment. With this system (2) downloaded in a user's device, it is like the user having a mobile pharmacy or a mobile clinic. In the system (2), a mobile app or an application act as the command center that issues instructions for the computer (31) or the smartphone (32) to execute. This design allows seamless combination with AI medical and diagnosis systems and/or AI robots to provide more accurate mobile medical services for human beings or other living organisms.

## Description

### BACKGROUND OF THE INVENTION

### 1. Field of the Invention

This disclosure relates to a system for activation of cells remotely according to the preamble of claim 1. Accordingly, the present invention severally relates to biomedical sciences and more particularly, to a system that activates living cells remotely and an application method of the system.

### 2. Description of the Related Art

Bionic technology has been used in various fields to improve human life and medical treatment, including robots having bionic forms and motions, and materials featuring high-quality bionic performance. However, there has never been any product or digital electronics that really work biologically for biomedical purposes. Also, there has never been any bionic product or technique that is really effective in carbon reduction and environmental friendliness. Most existing bionic systems, before reaching their users, have to be manufactured in factories and then shipped through transportation systems, and usually end up being discarded only after a few times of use.

This quantum theory has exhibited its potential application value in various scientific fields. The quantum entanglement phenomenon has provided a whole new way to transmit information. This brings about new possibilities for remote activation of cells. At present, cell activation depends mainly on physical or chemical means, but it has issues like low efficiency, poor versatility, and the need for treatment in a clinic or hospital.

According to research, there is about 30 to 40% doctors' prescriptions for patients with asthma or diabetes which did not result in curative effects as expected. The number increases to 50 to 75% when it comes to patients with arthritis or Alzheimer's Disease. The underlying cause is that when a medicine shows curative effects in most subjects with similar conditions in its human subject research, it would traditionally be approved to be used in patients with these conditions. However, what about the patients experiencing low or no efficacy who are ignored? In other words, the conventional approach to medicine development is for the average person. In fact, however, each of us and the illnesses we have are unique. Many medicines may not be effective for all patients, and in the worst case, they may cause serious adverse effects. As we recognize more about genetic differences among individuals, it is believed that tailored, personalized medicine is the best solution.

Source: "Tailor-made precision medicine" by Tom Ireland, BBC Knowledge, Vol. 80, Apr. 2018.

It is known that the pharmaceutical industry is responsible for environmental pollution in many aspects, including material acquisition, plant cultivation, animal extraction, chemical synthesis, product packaging and transportation. Another proof that this industry is causing significant impact is its global carbon emission. According to research, the pharmaceutical industry emits 55% more carbon than the automobile manufacturing industry, despite its smaller market share, which is only 28% globally. The detailed data of carbon emission and sources are provided below:
Source: "How can the pharmaceutical sector reduce its carbon footprint?" by Alf Goebel, CEO of Advanco, Aug. 25, 2021.

### Materials acquisition:

1. Chemical synthesis: Production of medicines usually involves the use of a large amount of chemicals. These chemicals may escape to the surroundings, including both air and adjacent bodies of water, during production. These chemicals may include organic solvents, heavy metals, and other poisonous substances.
2. Plant cultivation: Plants for pharmaceutical use are usually cultivated with large amounts of pesticides and fertilizers. These substances can leach into soil and waters and cause agricultural pollution.

### Production:

1. Chemical synthesis and pharmaceutical processes: These processes consume substantial energy, particularly electricity and heat that result from burning fossil fuels, leading to high carbon emissions.
2. API production: During production of active pharmaceutical ingredients (APIs), large amounts of pollutants are released, and this further increases carbon emissions.

### Transportation and supply chain:

The global pharmaceutical supply chain, including transportation from production sites to all corners of the world, burns considerable fossil fuels and causes significant carbon emissions. As reported, emissions throughout the supply chain account for more than 80% of total pharmaceutical emissions.

### Waste treatment:

Treatment of wastewater and waste: As a part of pharmaceutical production, treatment of wastewater and solid waste also consumes a substantial amount of energy and further increases carbon emissions.

### The reasons for implementing the disclosed system in computers and smartphones:

Technical innovations should only be made to improve our lives, but not create more technical waste that aggravates climate warming.

A team of scientists led by Johan Rockström, a Swedish scientist born on Dec. 31, 1965, first introduced the concept of planetary boundaries in 2009, which was then updated in 2015.

As indicated by this team, our top priority is to halve global carbon emissions by 2030 and reach net zero by 2050. This means that all systems related to life must be of zero emission.

However, every time we buy a new electric appliance, it comes with different cables and accessories. When the appliance is updated or upgraded, more cables and accessories are included. People always have numerous connection cables and a pile of discarded devices in their houses. These high-tech wastes, which are continuously produced and recycled just for limited usage, are all killers to the planet.

If the environment we live in increasingly deteriorates, it is anticipated that a shortage of physical medicines will happen and make it difficult to deal with pandemic outbreaks that could occur every 10 years. Hence, we look to the biomedical community to keep developing a more effective remote approach to cell activation that minimizes physical contact between doctors and patients and reduces transportation costs. It is desired that such an approach allows cell activation carrier information corresponding to pandemics to be communicated to the whole world, for global users to download and use.

Especially, after the outbreak of COVID, we are desirous that medical technology develops toward production of remote digital electronic medicines, remote transmission of digital electronic medicines to patients, and development of digital electronic vaccines.

In the prior art, cell activation mainly depends on physical contact or chemical stimulation, but these require patients being present and could lead to potential harm.

In the prior art, for information transmission, at least a transmitter and a receiver are required. If information is to be transmitted to a human body, an animal, or a plant, the receiver has to be connected to or installed on the human body, the animal, or the plant.

In the prior art, supplies for cell activation, such as medicines, are only for single use and require continuous manufacturing, making them costly and environmentally unfriendly.

### SUMMARY OF THE INVENTION

To address foregoing issues, this disclosure provides a remote cell-activation system and its application method based on the quantum theory. The system may be implemented as a mobile app executable in a smartphone or an application executable in a computer, and features easy operation, high efficiency, and good versatility.

This disclosure provides a remote cell-activation system, which is configured to be installed in a computer or a smartphone. It comprises a target object's quasi-like quantum entanglement pair generating unit, a quasi-like quantum entanglement pair frequency-modulating unit, a quasi-like quantum entanglement pair executing unit, and a quasi-like quantum entanglement pair transmitting unit. The target object's quasi-like quantum entanglement pair generating unit uses the computer or the smartphone to acquire organism cell parameters which generate quasi-like quantum entanglement pairs. The quasi-like quantum entanglement pair frequency-modulating unit has a frequency-modulating element. The frequency-modulating element is used to generate cell activation carrier digital data and target object's quasi-like quantum entanglement pair digital data. The quasi-like quantum entanglement pair executing unit is used to resonate the cell activation carrier digital data with the target object's quasi-like quantum entanglement pair digital data so as to generate a waveform with a specific frequency. The quasi-like quantum entanglement pair transmitting unit is used to remotely transmit the waveform with the specific frequency generated by resonating the cell activation carrier digital data with the target object's quasi-like quantum entanglement pair digital data so as to activate cells in the physical target object.

This disclosed remote cell-activation system is related to biomedicine, and is a mobile app to be installed in a smartphone and an application to be installed in a computer. With the digital electronic technology of the smartphone or the computer, bionic targeting is performed on a target object. This includes obtaining the target object's quasi-like quantum entanglement pair digital data by photographing or voice recording performed using the lens and the recording device of the smartphone or the computer. The target object is a living organism. According to the type of target object relevant cell activation carrier digital data are selected from those pre-programmed in the system for optimization and the electric fields are used for cell activation. A user may add suitable cell activation carrier digital data according to the target object's needs. The addition can then be sent as cell activation carrier digital data to the target object through the computer or the smartphone by the advantage of quantum entanglement effects, thereby eliminating the need for contacting the target object and being free of distance limitation.

This disclosure provides a remote cell-activation system and its application method. The remote cell-activation system uses the digital electronic technology to perform bionic targeting on a living organism so as to obtain the quasi-like quantum entanglement pair digital data. The bionic mechanism of the remote cell-activation system is about obtaining the target object's quasi-like quantum entanglement pair digital data by photographing the target object's images and recording the target object's voice. A user may add relevant cell activation carriers according to different needs for cell activation. At last, through the computer or the smartphone, remote activation of cells can be accomplished utilizing quantum entanglement effects. During the process, the target object does not need to wear any message-receiving equipment or connect to the internet.

This disclosure, based on quantum entanglement effects, provides a remote cell-activation system and its application method to break through limits seen in the prior art.

This disclosure breaks through the prior art to allow the target object to be free from any need to wear or connect any message-receiving equipment.

In this disclosure, the cell activation carriers can be reused forever, making the system favorable to environmental protection, resource preservation, and sustainability.

This disclosure provides a novel remote cell-activation system. The system uses quasi-like quantum entanglement pairs to realize remote transmission of information and incorporates a dynamic carrier database to allow customization of cell activation schemes tailored for users' needs. This disclosure not only reduces the need for long-haul transportation, but also helps decrease overall carbon emission, and thus has a wide range of applications and huge market potential.

The sound of the target object is recorded, and then the sound that is played is the quasi-like quantum entanglement pair of the target object.

The image of the target object is photographed and then the image presented is the quasi-like quantum entanglement pair of the target object.

For example, my voice played by a computer or mobile phone is my quasi-like quantum entanglement pair, and my image presented by a computer or mobile phone is my quasi-like quantum entanglement pair. The image of a certain medicinal material presented by a computer or mobile phone is the quasi-like quantum entanglement pair of the medicinal material, and the image of the plant presented by a computer or mobile phone is the quasi-like quantum entanglement pair of the plant.

Although the digitized images and sounds of the target object are not exactly the same as the physical target object, the photos of the physical target object and the target object look similar, and the sound played by the device sounds similar as emitted by the physical target object. Although the digitized image and sound of the target object are not equal to the physical target object they are similar in nature; one is an entity, and the other is the digitization of the entity, so I call them "quasi-like quantum entanglement pairs", and the photo files and sound files of the target object are called "quasi-like quantum entanglement digital data/information."

### BRIEF DESCRIPTION OF THE DRAWINGS

FIG. 1 is a block diagram showing application architecture of a remote cell-activation system according to this disclosure.
**FIG. 2** is a flowchart illustrating extraction of quasi-like quantum entanglement pair digital data according to this disclosure.
**FIG. 3** is a flowchart illustrating selection and application of cell activation carriers according to this disclosure.
**FIG. 4** is a schematic drawing of the computer-version operational interface of the disclosed system.
**FIG. 5** is a schematic drawing of the smartphone version operational interface of the disclosed system.
**FIG. 6** shows movement of plasma in a computer display (screen) for displaying pictures according to this disclosure.
**FIG. 7** illustrates electric fields generated when the system of this disclosure operates.
**FIG. 8** is a plane view of the executed mobile app and application according to the disclosed system.
**FIG. 9** is a lateral view of the executed mobile app and application according to the disclosed system.
**FIG. 10** shows a screen in the computer version of the disclosed system, wherein areas of cell activation carrier digital images and targets requiring cell activation are displayed.
**FIG. 11** illustrates current variation in electric fields in a dynamic meridian system in the disclosed system.
**FIG. 12** shows a screen in the computer version of the disclosed system, wherein cell activation carrier digital images are superposed as layers.
**FIG. 13** shows transmission between a parent electric field and a child electric field through quasi-like quantum entanglement pairs according to this disclosure.
**FIG. 14** shows that when a computer or a smartphone with the disclosed system is powered on, an electromagnetic field A is formed.
**FIG. 15** shows that when the mobile app or the application of the cell-activation system of this disclosure starts to operate, an electromagnetic field B is formed.
**FIG. 16** shows that when a target object's voice or images continuously operate, an electromagnetic field C is formed.
**FIG. 17** shows execution schematic in the smartphone-version mobile app of this disclosure.

### DETAILED DESCRIPTION OF THE PREFERRED EMBODIMENT

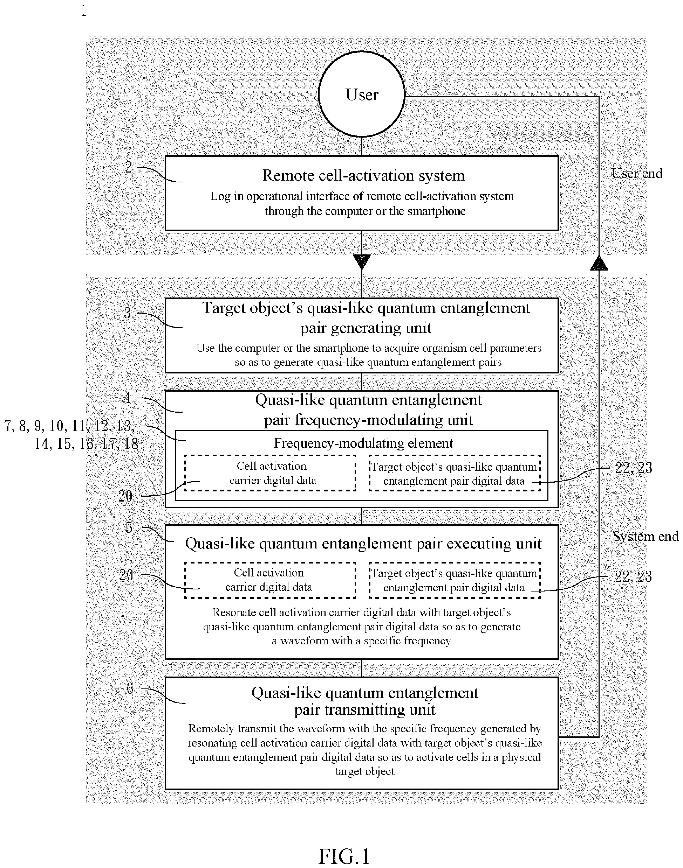

Referring to **FIG. 1****,** this disclosure provides a remote cell-activation system **2,** which is configured to be installed in a computer **31** or a smartphone **32.** A user may log in to the remote cell-activation system **2** through its operational interface. The remote cell-activation system **2** comprises a target object's quasi-like quantum entanglement pair generating unit **3,** a quasi-like quantum entanglement pair frequency-modulating unit **4,** a quasi-like quantum entanglement pair executing unit **5,** and a quasi-like quantum entanglement pair transmitting unit **6.** The target object's quasi-like quantum entanglement pair generating unit **3** uses the computer **31** or the smartphone **32** to acquire organism cell parameters so as to generate quasi-like quantum entanglement pairs. The quasi-like quantum entanglement pair frequency-modulating unit **4** has frequency-modulating elements **7~18**. The frequency-modulating elements **7~18** are used to generate cell activation carrier digital data **20** and target object's quasi-like quantum entanglement pair digital data **22, 23.** The quasi-like quantum entanglement pair executing unit **5** is used to resonate the cell activation carrier digital data **20** with the target object's quasi-like quantum entanglement pair digital data **22, 23** so as to generate a waveform with a specific frequency. The quasi-like quantum entanglement pair transmitting unit **6** is used to remotely transmit the waveform with the specific frequency generated by resonating the cell activation carrier digital data **20** with the target object's quasi-like quantum entanglement pair digital data **22, 23** so as to activate cells in a physical target object **21.** The target object **21** is a living organism.

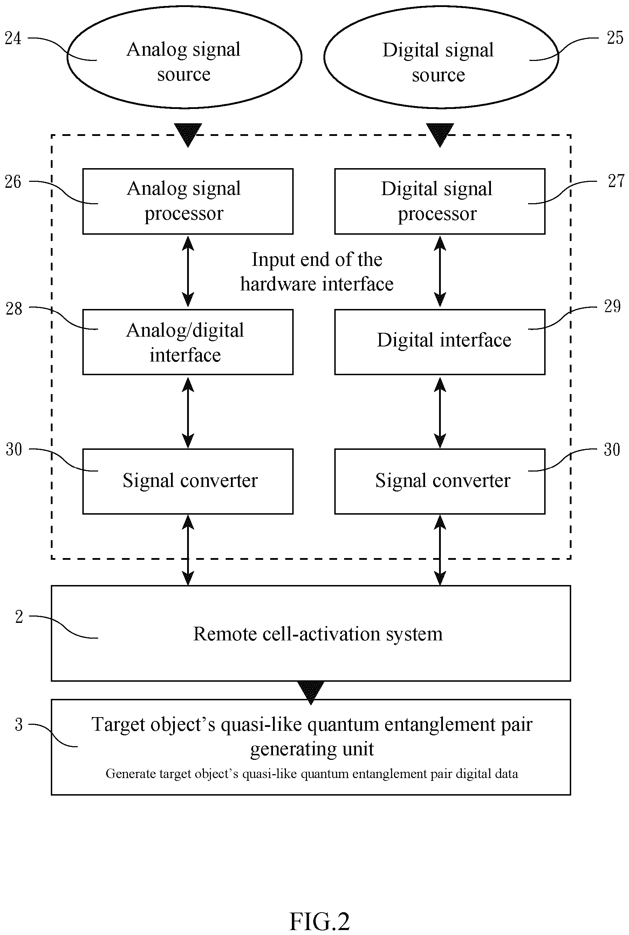

Referring to **FIG. 2****,** the disclosed remote cell-activation system **2** comprises an analog signal source **24** and a digital signal source **25.** The analog signal source **24** is connected to an analog signal processor **26** for signal processing. The processed signal is input to an analog/digital interface **28** at the input end of the hardware interface. The digital signal source **25** sends a signal to a digital signal processor **27** for processing. The processed signal is then input to a digital interface **29** at the input end of the hardware interface. The signals processed by the analog/digital interface **28** or the digital interface **29** then enter a signal converter **30.** The signal converter **30** converts the signals in different format into the same digital signal format before entering them to the remote cell-activation system **2.** The remote cell-activation system **2** is configured to execute a logical application related to cell activation or other biological techniques to thereby generate the target object's quasi-like quantum entanglement pair digital data **22, 23.**

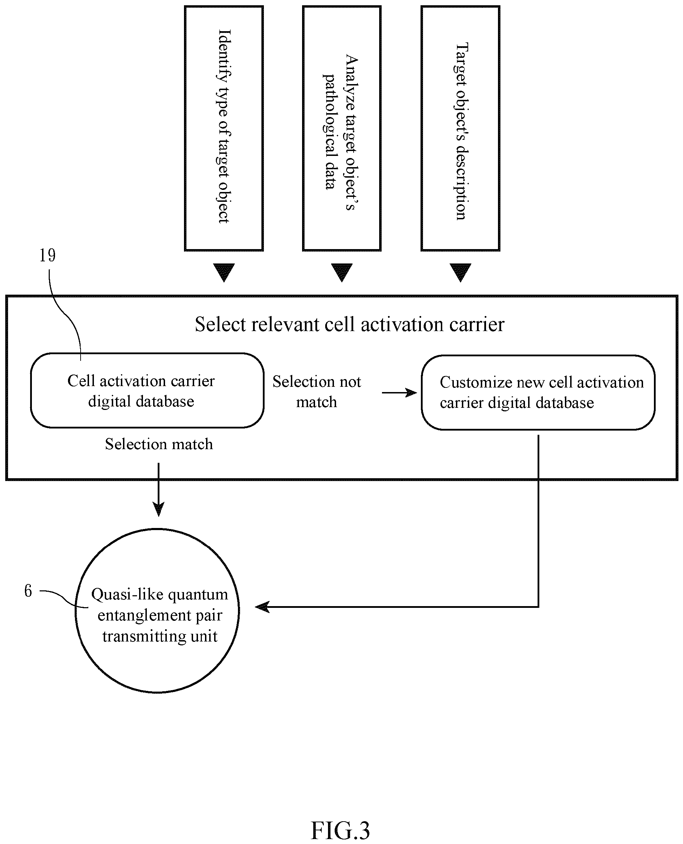

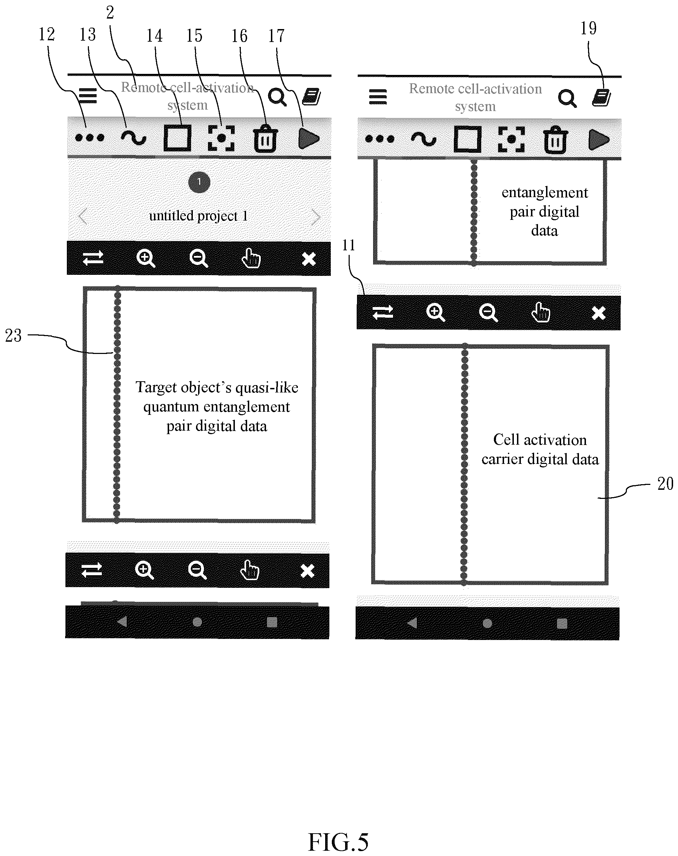

Referring to **FIG. 3** through **FIG. 5** and **FIG. 13****,** selection of the relevant cell activation carriers is now explained. First, a user identifies and analyzes the target object **21** by classifying the target object **21** and analyzing pathological data of the target object **21** and description of conditions made by the target object **21.** This is to determine the specific attributes of the target object **21** to enable an informed selection of the most relevant cell activation carriers. With the analysis results related to the target object **21,** the user then performs the step of "selecting the relevant cell activation carriers". At this step, the remote cell-activation system **2** first accesses a cell activation carrier digital database **19,** which contains various kinds of the cell activation carrier digital data **20,** so that the user can search for the cell activation carrier digital data **20** matching the illness of the target object **21** from the cell activation carrier digital database **19.** If there are cell activation carrier digital data **20** matching the target object **21** in the cell activation carrier digital database **19,** the user selects these cell activation carrier digital data **20** for subsequent operations. Otherwise, the user has the option to customize new cell activation carrier digital data **20** that match the illness attributes of the target object **21.** With the selected or customized cell activation carrier digital data **20,** the remote cell-activation system **2** operates the quasi-like quantum entanglement pair transmitting unit **6** to start the process of cell activation, in which the selected or customized cell activation carrier digital data **20** is applied to treat the target object **21.**

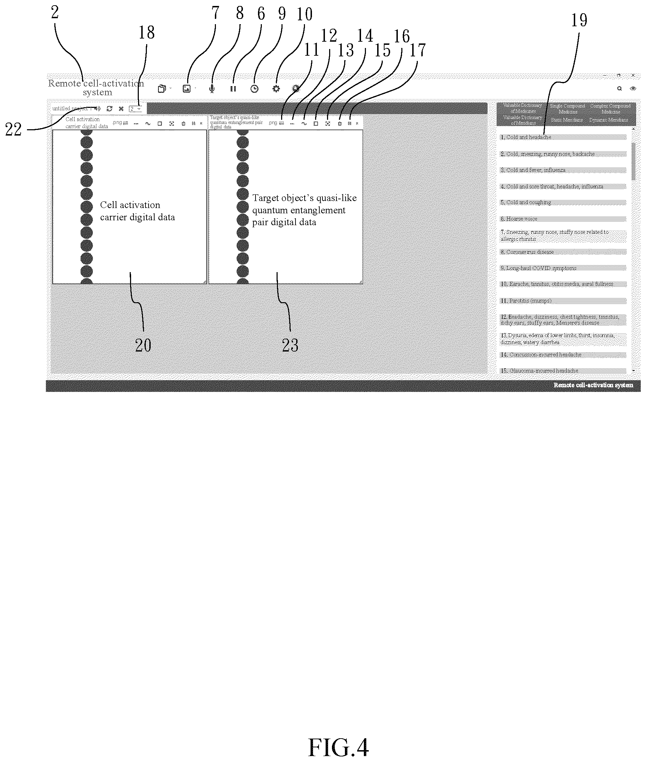

Still referring to **FIG. 4** through **FIG. 10****,** in an area of the cell activation carrier digital data **20** and in an area of the target object's quasi-like quantum entanglement pair digital data **22,** inherent frequency thereof is enhanced by making a plurality of digital red dots **34** which continuously flash or scan. The digital red dots **34** continuously flashing indicate moving targets on a meridian diagram and an anatomical diagram in order to facilitate enhancement of activity in the electric field of the corresponding areas.

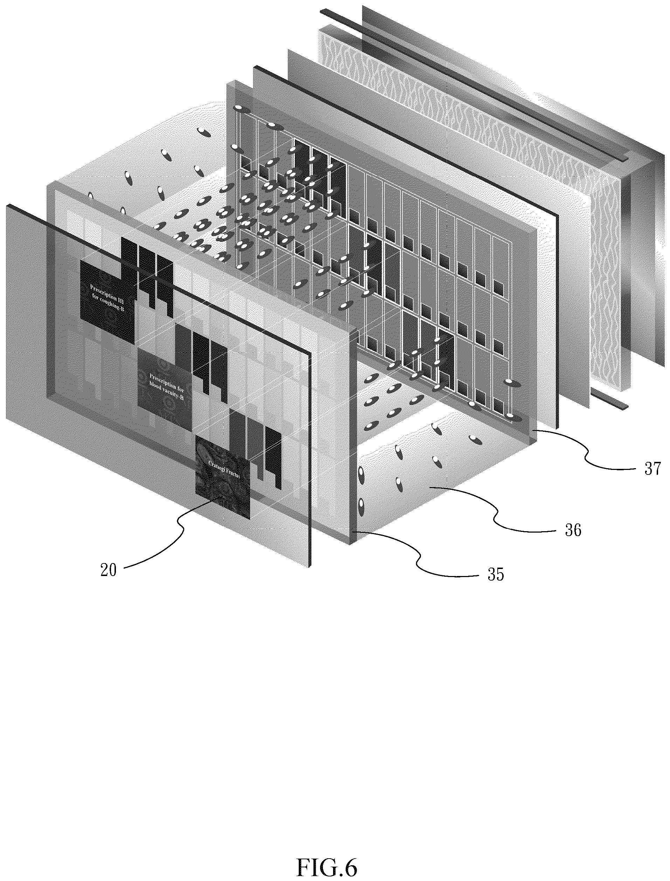

Referring to **FIG. 6** through **FIG. 17** along with **FIG. 1** through **FIG. 5****,** implementation of this disclosure can be divided into two parts:
1. Software: a mobile app and an application
2. Hardware: a smartphone **32** and a computer **31**

Part One: Software: the mobile app and the application

There are two ways to extract the target object's quasi-like quantum entanglement pair digital data **22, 23:**
i. Photographing images: the user uses lens installed on the computer **31** or the smartphone **32** to acquire image data of the target object **21.** The image data is then converted into the target object's quasi-like quantum entanglement pair digital data **22, 23,** which have frequency fluctuations similar to those of the target object **21.**
ii. Recording voice: the user uses the microphone provided on the computer **31** or the smartphone **32** to collect voice data of the target object **21.** The voice data is then converted into the target object's quasi-like quantum entanglement pair digital data **22, 23,** which have frequency fluctuations similar to those of the target object **21.**

Selection of the cell activation carrier digital data **20** is made with the possibility of adding more cell activation carrier digital data **20** that satisfy different requirements for activating different types of cells:
i. Determination is made:
   1. according to the type of the target object **21** as identified previously
   2. according to pathological analysis of the target object **21,** and/or
   3. according to the description made by the target object **21.**
ii. For flexible and full use of resources of the disclosed remote cell-activation system **2,** this disclosure has developed and provided the following features:
   1. The cell activation carrier digital database **19** has been designed to be updatable to meet varying requirements for cell activation.
   2. This disclosure allows selection of the relevant cell activation carrier digital data **20** from the pre-loaded cell activation carrier digital database **19** according to users' needs and types of cell activation.
   3. This disclosure provides for creating new, relevant cell activation carrier digital data **20** if no suitable cell activation carrier digital data **20** can be found in the cell activation carrier digital database **19.**

Software execution:
I. Remote control: The computer **31** or the smartphone **32** is used to ensure that the information of the cell activation carrier digital data **20** can be accurately transmitted to the target cells in the target object **21** by means of the quasi-like quantum entanglement pair technology.
II. Cell activation: The computer **31** or the smartphone **32** is used to perform operations of cell activation, which involve continuously transmitting the activation information to the target cells in the target object **21** by means of the quasi-like quantum entanglement pair technology.
III. The user makes further adjustments and optimizations according to performance of the resultant cell activation.

### Part Two: Hardware: the smartphone 32 and the computer 31

### Hardware execution:

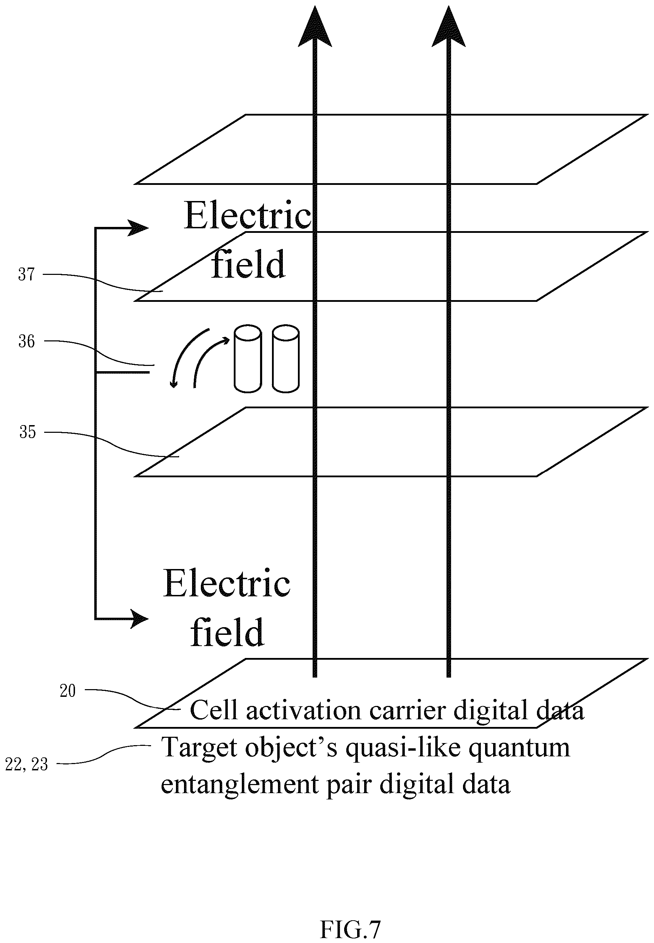

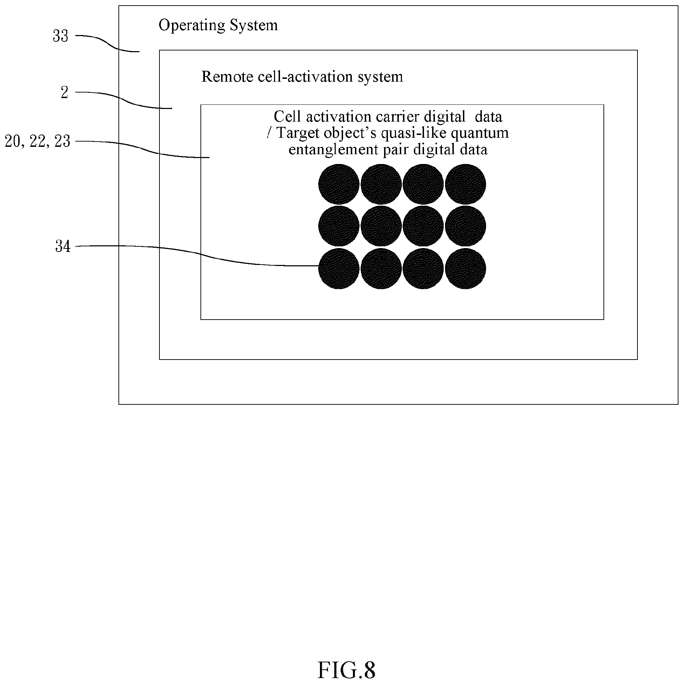

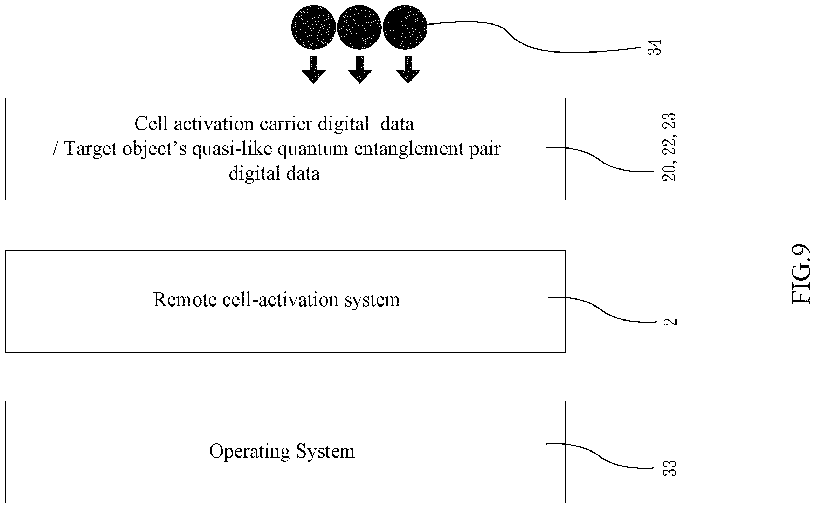

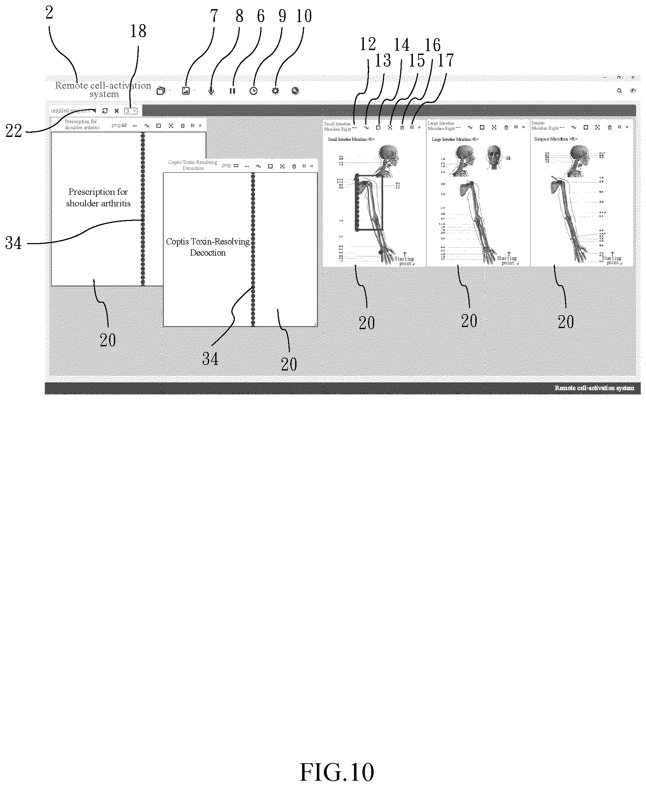

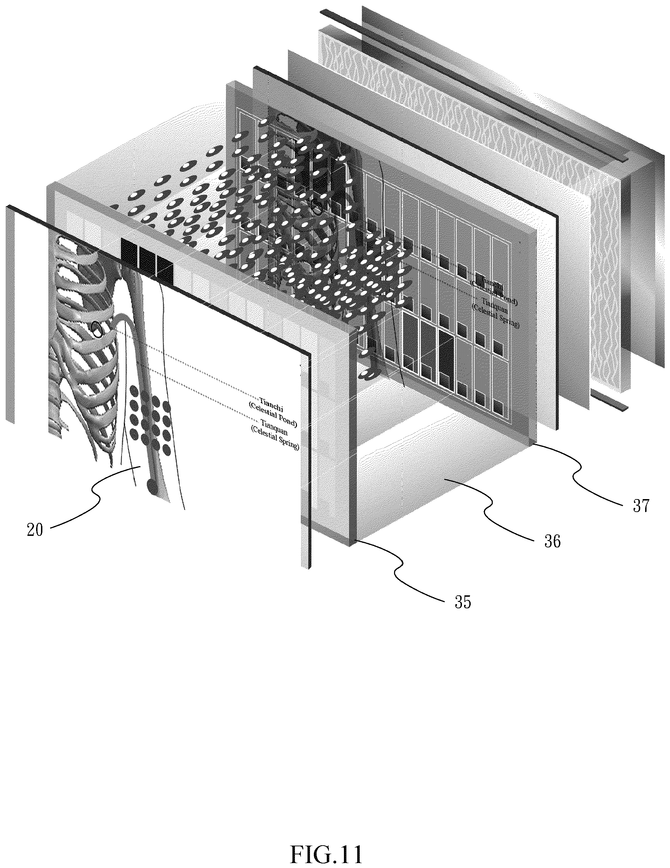

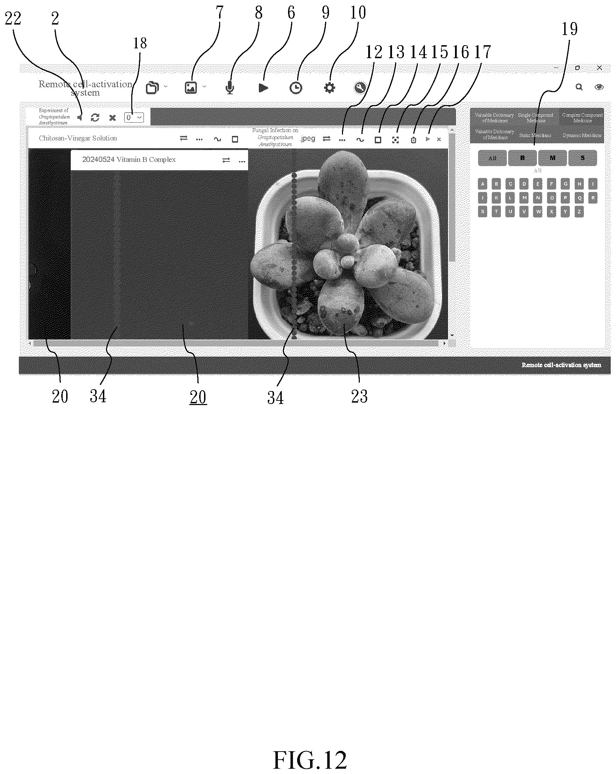

I. This disclosure uses the mobile app and the application to instruct the smartphone **32** and the computer **31** to execute tasks for cell activation, as described below:
   1. Electromagnetic waves can be measured around a powered computer **31,** and this fact indicates that the whole device forms an electric field that has expanded movements of electrons. On this basis, this disclosure provides the following controls and designs:
      (1) Referring to **FIG. 6** and **FIG. 7****,** frequent movements of electrons happen between the display and the hardware driver, and these electron movements generate an electromagnetic field. An electromagnetic field is generated when electrons flow between a front conductive glass **35** and a thin-film transistor **37.** Changes happening in the electric field can drive arrangements of liquid crystals within a liquid crystal electron movement zone **36.**
         Referring to **FIG. 8** and **FIG. 9****,** the disclosed remote cell-activation system **2** is a software program designed to run in an operating system **33** to perform scanning or tapping operations. By making full use of the electromagnetic field, multiple digital red dots **34** can be made to flash. This allows the digital red dots **34** to tap on pictures. Such "tapping" of the digital red dots **34** as perceived visually is in fact flashing of pixels in the form of round dots, which is achieved via high voltage, low voltage, and electron stacking. In order to maintain a stable electric field throughout this process and to continuously deliver resonant waves, the tapping made on the carriers has to be consistently maintained.
      (2) Referring to **FIG. 8** and **FIG. 9****,** in this disclosure, there are mainly two types of images involved, including the target object's quasi-like quantum entanglement pair digital data **22, 23,** and the cell activation carrier digital data **20.** Making the remote cell-activation system **2** scan/tap on the images is to enhance the inherent frequency of the images.
      (3) Referring to **FIG. 10****,** this disclosure is about activating movements of electrons in targeted sites on an anatomical diagram or a meridian diagram thereby increasing bonding and resonance between the targeted sites and voice. For example, in the event of injuries in the scapula and elbow joints, these areas may be marked on the anatomical diagram or the meridian diagram. Then with the cell activation carrier digital data **20** provided, tapping and scanning can be made by the digital red dots **34** to enhance the inherent frequency in these areas, thereby facilitating activation of cells in these areas.
      (4) Referring to **FIG. 11****,** this disclosure is further extended to have a dynamic meridian system and increase activities of electrons so as to achieve global cell activation in a living body.
      (5) Referring to **FIG. 12****,** this disclosure uses electron tunneling effects and electron sharing to allow selection of multiple kinds of the cell activation carrier digital data **20,** according to needs specific to the target object **21.** When the multiple kinds of cell activation carrier digital data **20** take up the whole screen, the images can be layered, and the second layer, the third layer, and so on up to the eighth layer are all as valid as the first layer. Particularly, a smartphone **32** usually has a relatively small screen area, so when images of multiple kinds of the cell activation carrier digital data **20** have to be executed, they can be overlapped. When the remote cell-activation system **2** operates, all subprograms related to this application continuously operate and react even if they are hidden as background processes. Superposition of electrons represents another concept. For example, a population of electrons exists in a digital image, and multiple digital images means that there are multiple populations of electrons. These electrons are not fixed to a certain picture; instead, they are shared among different images. These images can have their information therein converted toward each other when electrons share orbitals.
   2. There are two ways to transmit the cell activation carrier digital data **20** to the target object **21,** including:
      (1) through the quasi-like quantum entanglement pair digital data of the voice of the target object **21,** and
      (2) through the quasi-like quantum entanglement pair digital data of the images of the target object **21.**

This disclosure is based on quantum entanglement effects and provides carrier waves by continuously playing the voice of the target object **21** or continuously tapping or scanning images of the target object **21** so that fluctuations of the computer **31** or the smartphone **32** during resonance can be sent to the target object **21.** For the purpose of this disclosure, the voice may be played at any level of volume other than mute. Playing through earphones is also acceptable. In this process, the target object **21** does not have to hear the voice or see the contents on the screen of the computer **31** and the smartphone **32.**

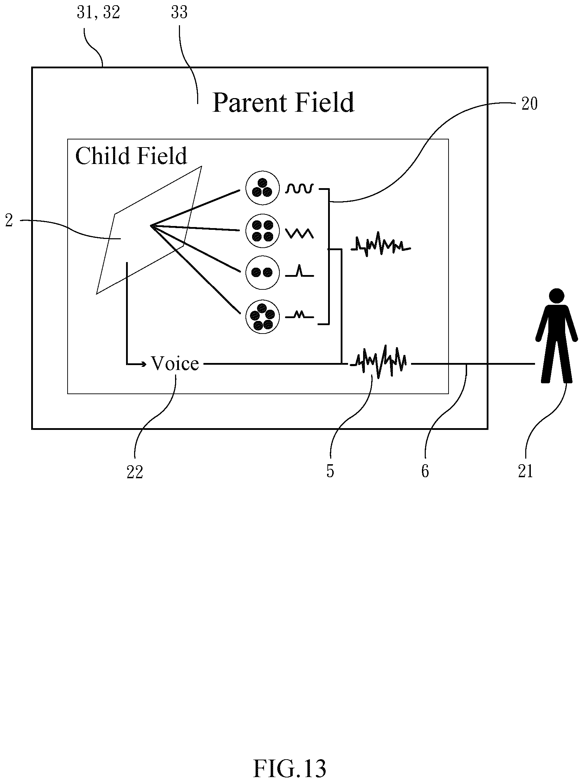

As shown in **FIG. 13****,** there are electron movements in digital pictures, and there are electron movements in displayed pictures. Tapping by the digital red dots **34** is not about physically contacting a picture. It is actually interaction between circuits of two different systems. With the electrons of the displayed picture and the electrons of the tapping digital red dots **34** both having continuous movements, an electric field can be formed. The active electric field can generate electromagnetic fields that expand in all directions. However, there is only one electromagnetic field fluctuating in the same way as the voice. In a mechanism so formed, a parent field is at the smartphone **32** or the computer **31,** and a child field is formed by the mobile app or the application, while the circles represent different digital pictures, each having their own fluctuations. This disclosure furnishes the fluctuation with meaning by introducing the voice or images of the target object **21.** They are in the same entanglement pair as the real-world target object **21** and have a similar fluctuation frequency to generate entanglement resonance. By continuously playing the digital voice of the target object **21** in the parent field or by continuously scanning the digital images of the target object **21** using the digital red dots **34,** the converted fluctuations can be transmitted to the target object **21** no matter how far the distance is.

3. The target object **21** can receive the information of the cell activation carrier digital data **20** without the need to wear or connect any devices. In this disclosure, all of the target objects **21,** which accept cell activation have electromagnetic reactions and can send and receive electronic signals. For example, a human body is an electrical conductor, which acts as an antenna. Although a human body and an antenna are different in terms of structure and composition, both have electrons moving therein. Ultrasound, X-ray, CT scan, MRI, etc. are all used to diagnose diseases by reading movements of electrons in a human body.

Specifically, a human body can, in fact, be regarded as an electronic transceiver, which sends and receives electronic signals. When receiving signals, a human body acts as an antenna, and when transmitting information, it acts as a radio station. Human voice is a kind of electromagnetic waves emitted by a human body. This disclosure is based on entanglement effects. Specifically, with the voice or image of the target object **21** that is also an entanglement pair, the target object **21** can be targeted for transmission of electronic signals. In this case, the human body or the plant/animal acting as the target object **21** can receive information without wearing or carrying any receiver.

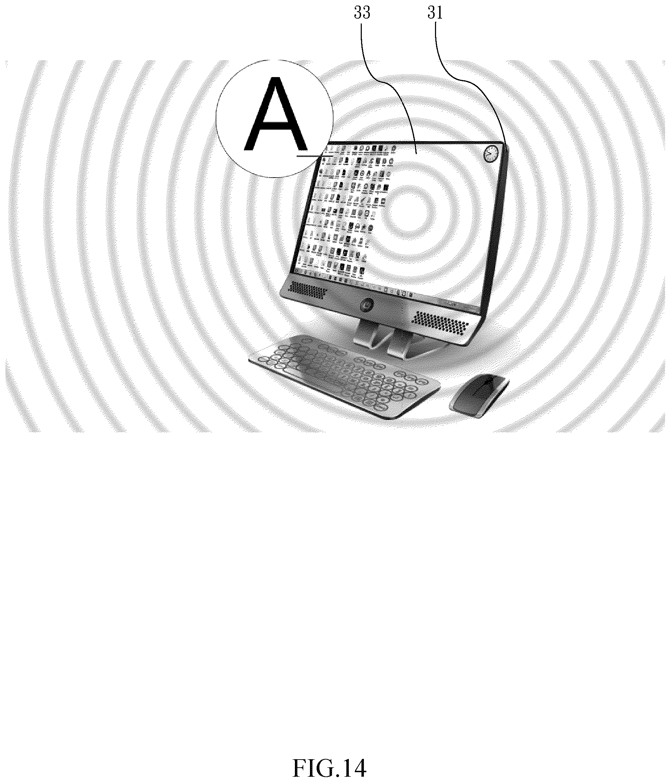

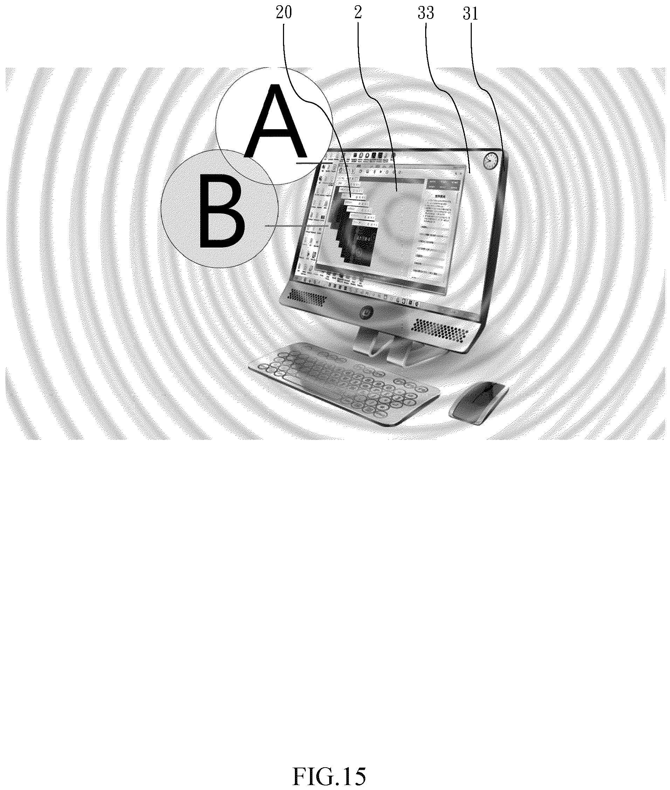

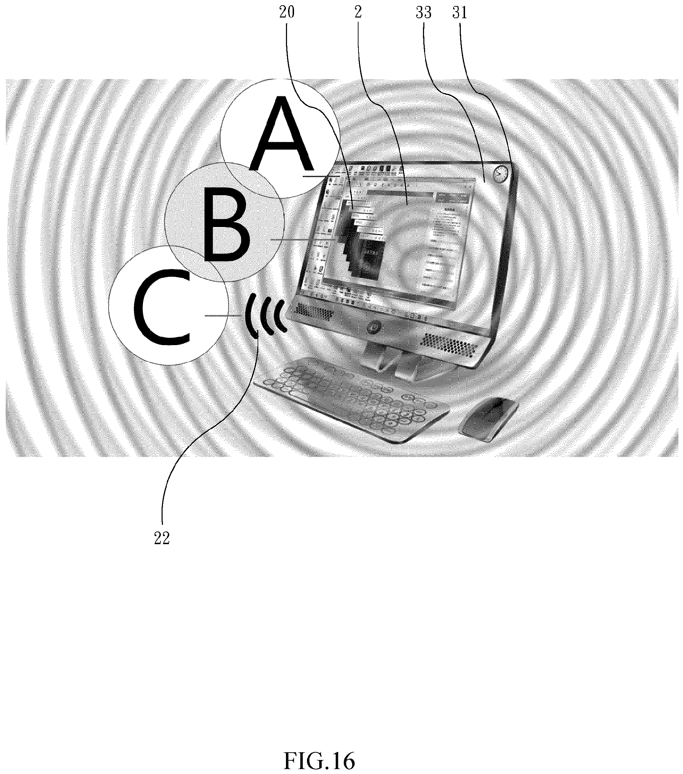

II. This disclosure uses a computer **31** and a smartphone **32** to produce cell activation carriers and electric fields for resonating the cell activation carrier, as detailed below:
1. Production of three electromagnetic fields.
   (1) Referring to **FIG. 14****,** when the computer **31** and the smartphone **32** are powered on, the computer **31** and the smartphone **32** form an electromagnetic field A. Within A, two more electromagnetic fields B and C are generated.
   (2) Referring to **FIG. 15****,** when the mobile app and the application of the remote cell-activation system **2** operate, the electromagnetic field B is generated.
   (3) Referring to **FIG. 16****,** when the voice or images of the target object **21** continuously operate, the electromagnetic field C is generated.
2. The three electromagnetic fields A, B, and C operate simultaneously.
   Referring to **FIG. 16****,** when the three electromagnetic fields A, B, and C operate at the same time, inter-field overlapping resonance happens. Such overlapping resonance activity is actually electronic activity because it is formed by a series of codes in digital units.
3. The information during the overlapping resonance period is transmitted to the target object **21.**
   The information is transmitted to the target object **21** mainly through making the computer **31** or the smartphone **32** play the voice or pictures of the target object **21.**

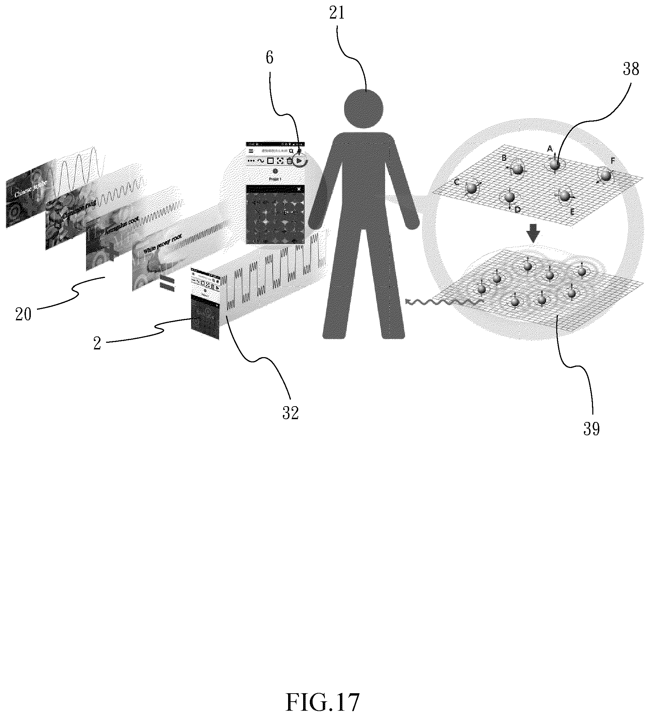

(1) In this disclosure, the digital voice of the target object **21** mainly serves to:
   a. Lock-on the target object **21** who is providing the voice. Since the voice from the target object **21** and the voice played by the computer **31** or the smartphone **32** are made by the same target object **21,** entanglement resonance can be achieved even if the computer **31** or the smartphone **32** playing the voice is far away from the target object **21.**
   b. Act as the carrier wave for transmitting the cell activation carrier fluctuations. All the carriers and voice in the electric field within the computer **31** or the smartphone **32** experience Fourier transformation, and every set of the resultant electron packets contains voice fluctuations. The wave of the voice is just like a conveyor belt that delivers the packages to the target object **21** providing the voice. Similarly, the images of the target object **21** can also be used as carrier waves.
(2) How to continuously enhance the entanglement effects of the entanglement pairs:
   In order to continuously enhance the entanglement effects of the entanglement pairs, the computer **31** and the smartphone **32** have to continuously play the voice or scan images of the target object **21** throughout the resonating process or the entanglement effects will cease. The voice can be adjusted to the minimum level of volume or be played through earphones, but mute is unacceptable. During this process, it is not necessary that the target object **21** hears the voice and sees the images displayed through the computer **31** or the smartphone **32.**
   4. The differences within the subject caused by the foregoing resonance and transmission will be discussed below.
   (1) Referring to **FIG. 17****,** when a human body or an animal or a plant is ill, the atomic activity becomes irregular **38,** meaning that atoms behave disorderly and cells are weak.
   (2) Cell activation is made to facilitate recovery of health. Still referring to **FIG. 17****,** since the carrier fluctuations output by the computer **31** or the smartphone **32** are stable, when particles of these carrier fluctuations hit on particles inside the target object **21,** resonance happens between them, which leads to harmonic oscillation. Such harmonic oscillation can gradually modulate irregular fluctuations in the target object **21** caused by diseases to turn them into stable and orderly fluctuations and achieve regular atomic activity **39,** thereby accomplishing cell activation.

A human, an animal, or a plant which is ill may be too passivated and frail to trigger an immune response against bacteria. The remote cell-activation system **2** can facilitate cell activation and enable the target object **21** to fight germs. An operator can select or add suitable cell activation carrier digital data **20** for cell activation according to illness and conditions of the target object **21.**

### Description of embodiments:

i. Images or voice is captured and recorded using the lens or recording device of the computer **31.** The target object's quasi-like quantum entanglement pair digital data **22, 23** are acquired through the application. The cell activation carrier digital data **20** suitable for activation of immunocytes are selected and remotely transmitted to cells in the target object **21** for activating the cells remotely.
ii. Images or voice is captured and recorded using the lens or recording device of the smartphone **32.** The target object's quasi-like quantum entanglement pair digital data **22, 23** are acquired through the mobile app. The cell activation carrier digital data **20** suitable for restoration of histiocytes are selected and remotely transmitted to cells in the target object **21** for restoration.

{Embodiment 1}: when a target object **21** needs remote activation of skin cells, a user instructs a smartphone **32** to capture images of the skin of the target object **21** and record the voice of the target object **21** through the remote cell-activation system **2.** Then the user selects suitable cell activation carrier digital data **20,** and transmits the cell activation carrier digital data **20** to the skin cells of the target object **21** remotely by means of the quasi-like quantum entanglement pair technology, thereby activating the skin cells remotely.

{Embodiment 2}: In an agricultural scenario, when it is desired to activate cells of plants remotely, images of the plants are captured and sent to the remote cell-activation system **2.** According to the images, the reason why cells of the plants are frail is determined. Then suitable chemical molecule digital data are selected as the cell activation carrier digital data 20, and remotely transmitted to the cells of the plants by means of the quantum entanglement pair technology for activating the cells remotely.

[Experiment example 1]: In one gardening farm, plants were suffering from fungal infection. An experimental plant was measured for its bacterial count before the experiment and the bacterial count was 8126. After 5 days of activation of cells by means of remote chitosan-vinegar solution digital carrier resonance (6h per day), the bacterial count decreased to 6911. After 5 more days of resonance, the measured bacterial count was 13967, and the plant was stronger than 10 days ago. On the 15^{th} day, the measured bacterial count was 20051. The plant with fungal infection would gradually decay and eventually die if it were left untreated. The plant showed an increase in bacterial count but became stronger than 15 days ago. This is because good bacteria and bad bacteria in the plant both had wax and wane. In the experiment, when a plant was visually recognized as growing stronger, it was determined that the plant had its cells activated and the good bacteria therein increased to support immunity against the bad bacteria. This experiment was performed to prove that with the right cell activation carriers, an ill plant could be protected from aggravation of conditions and restore its health gradually.

**[Table 1]**

| | Bacterial count measured before experimen t | Resonanc e w. chitosan-vinegar solution digital carriers-Bacterial count measured on the 5^{th} day (6h per day) | Resonanc e w. chitosan-vinegar solution digital carriers-Bacterial count measured on the 10^{th} day (6h per day) | Resonanc e w. chitosan-vinegar solution digital carriers-Bacterial count measured on the 15^{th} day (6h per day) |
|---|---|---|---|---|
| Experimenta 1 plant | 8126 | 6911 | 13967 | 20051 |

**[Table 2]**

| | Resonance w. chitosan-vinegar solution digital carriers-Bacterial count measured on the 23^{rd} day (3h per day) | Resonance w. chitosan-vinegar solution digital carriers + 1:5 diluted solution of Vit. B complex carrier - Bacterial count measured on the 29^{th} day (3h per day) | Resonance w. chitosan-vinegar solution digital carriers + 1:5 diluted solution of Vit. B complex - Bacterial count measured on the 38^{th} day (3h per day) |
|---|---|---|---|
| Experimental plant | 8969 | 11495 | 14159 |

Simply watering the plants with clean water during the experiment without providing any nutriment or fertilizer might be unfavorable to a proliferation of good bacteria. The experiment thus additionally used the Vitamin B complex carriers for resonance. While these carriers did not supply nutriment directly, they provided fluctuations for activating good bacteria. As observed, the plants so treated remained strong and put forth new leaf layers.

### [Experiment example 2]

1. The first experiment: Liquid kitchen waste was used **in** the experimental group by adding 10cc of liquid kitchen waste in each cell-culture dish and resonating the cultures with 10cc liquid bleach.

**[Table 3]**

| | Bacterial count measured before experiment | Resonance w. 10cc bleach-Bacterial count measured after 1h | Resonance w. 10cc bleach-Bacterial count measured after 2h |
|---|---|---|---|
| Control | 67 | 86 | 47 |
| Experimental | 23 | 50 | 125 |

2. The second experiment: Liquid kitchen waste was used in the experimental group by adding 10cc of liquid kitchen waste in each cell-culture dish and resonating the cultures with 600cc liquid bleach.

**[Table 4]**

| | Bacteri al count measur ed before experi ment | Reson ance w. 600cc bleach Bacter ial count measur ed after 1h | Reson ance w. 600cc bleach Bacter ial count measur ed after 2h | Reson ance w. 600cc bleach Bacter ial count measur ed after 3h | Reson ance w. 600cc bleach Bacter ial count measur ed after 4h | Reson ance w. 600cc bleach Bacter ial count measur ed after 5h |
|---|---|---|---|---|---|---|
| Control | 3 | 3 | 9 | 4 | 13 | 4 |
| Experim ental | 3 | 6 | 22 | 13 | 12 | 10 |

This experiment has proven that:
(1) The remote cell-activation system 2 ensured safety of the living organism even with the use of bleach digital carriers in a huge amount.
(2) The bacterial count in the experimental group was greater than that of the control group because of targeted resonance with the experimental group. Even in the harsh environment (with high-concentration bleach), cells in the experimental group were still activated to have mass replication.
3. The third experiment: Liquid kitchen waste was used in the experimental group by adding 20cc of liquid kitchen waste in each cell-culture dish and resonating the cultures with a 1:5 diluted solution of an agricultural Vitamin B complex that is favorable to proliferation of composting bacteria.

**[Table 5]**

| | Bacteri al count measur ed before experi ment | Reson ance w. Vitami n B compl ex-Bacter ial count measur ed after 1h | Reson ance w. Vitami n B compl ex-Bacter ial count measur ed after 2h | Reson ance w. Vitami n B compl ex-Bacter ial count measur ed after 3h | Reson ance w. Vitami n B compl ex-Bacter ial count measur ed after 4h | Reson ance w. Vitami n B compl ex-Bacter ial count measur ed after 5h |
|---|---|---|---|---|---|---|
| Control | 2 | 9 | 4 | 4 | 7 | 2 |
| Experim ental | 3 | 29 | 5 | 5 | 4 | 5 |

This experiment has proven that:
(1) provision of suitable cell activation carriers is helpful for cell activation.
(2) The experimental group had a greater bacterial count as a result of cell activation as compared to the control group.
(3) Without provision of food in both cases, bacteria in the cultures gradually declined and eventually died after proliferation.

## Claims

1. A remote cell-activation system (2), which is configured to be installed in a computer (31) or a smartphone (32), the remote cell-activation system (2) **characterized by** comprising:
a target object's quasi-like quantum entanglement pair generating unit (3), which uses the computer (31) or the smartphone (32) to acquire organism cell parameters so as to generate quasi-like quantum entanglement pairs;
a quasi-like quantum entanglement pair frequency-modulating unit (4), which has a frequency-modulating element (7), the frequency-modulating element (7) being used to generate cell activation carrier digital data (20) and target object's quasi-like quantum entanglement pair digital data (22);
a quasi-like quantum entanglement pair executing unit (5), which resonates the cell activation carrier digital data (20) with the target object's quasi-like quantum entanglement pair digital data (22) so as to generate a waveform with a specific frequency; and
a quasi-like quantum entanglement pair transmitting unit (6), which is used to remotely transmit the waveform with the specific frequency generated by resonating the cell activation carrier digital data (20) with the target object's quasi-like quantum entanglement pair digital data (22) so as to activate cells **in** a physical target object (21).

2. An application method of a remote cell-activation system, **characterized by** comprising:
using digital electronic technology implemented in **a** smartphone (32) or a computer (31) to perform bionic targeting on a target object (21), which includes extraction of quasi-like quantum entanglement pairs from image and voice information of the target object (21), extraction of cell activation carrier digital data (20), post-extraction selection and optimization, application technology of electric fields, and remote execution and transmission based on quasi-like quantum entanglement pair technology for cell activation;
adding more cell activation carrier digital data (20) suitable for different needs for cell activation, and sending information of the cell activation carrier digital data (20) to the target object (21) through the computer (31) or the smartphone (32), wherein the target object (21) is free of any need for any message-receiving equipment and any internet connection, in which
the extracted image and voice information and the cell activation carrier digital data (20) are reusable repeatedly and permanently.

3. The application method of claim 2, **characterized by** comprising using the computer (31) or the smartphone (32) as an electric field for emitting cell activation carriers, and remotely transmitting and receiving digital information based on quantum entanglement effects as well as phenomena of superposition and sharing of electrons, so that the target object (21) is free from any need to wear any specialized equipment or connect with any connection line of any type.

4. The application method of claim 2, **characterized in that** carriers made of the cell activation carrier digital data (20) are reusable with unlimited time and unlimited distance, and that digital data made of the quasi-like quantum entanglement pairs of the information of the target object (21) are reusable with unlimited time and unlimited distance.

5. The application method of claim 2, **characterized in** technology by which the cell activation carrier digital data (20) made of a living body or matter is reusable with unlimited time and unlimited distance.

6. The application method of claim 2, **characterized in** technology in which quantum entanglement effects are used to capture images of or record voice of the target object (21) and to remotely transmit the cell activation carrier digital data (20) to the target object (21).

7. The application method of claim 2, **characterized in** selecting and optimizing image applications and operational technology for the cell activation carrier digital data (20).

8. The application method of claim 2, **characterized in** technology by which the cell activation carrier digital data (20) are sent to the target object (21) related to the quasi-like quantum entanglement pairs.

9. The application method of claim 2, **characterized in** technology by which three electric fields are generated to convert fluctuation of the cell activation carrier digital data (20).

10. The application method of claim 9, **characterized in** technology by which after the three electric fields A, B, and C are activated simultaneously, the electric field C of the quasi-like quantum entanglement pairs of the target object (21) is able to emit directional wave information while tracking movement of the target object (21).

11. The application method of claim 2, **characterized in** technology by which in an area of the cell activation carrier digital data (20) and an area of target object's quasi-like quantum entanglement pair digital data (22), inherent frequency thereof is enhanced by making a plurality of digital red dots (34) continuously flash or scan.

12. The application method of claim 11, **characterized in** technology by which the digital red dots (34) continuously flashing indicate moving targets on a meridian diagram and an anatomical diagram so as to facilitate enhancement of activity in the electric field of the corresponding area.
